# EUROPÄISCHE PATENTSCHRIFT

(11) **EP 3 481 350 B1**
(45) Veröffentlichungstag und Bekanntmachung des Hinweises auf die Patenterteilung: **14.04.2021**
(21) Anmeldenummer: 17728832.1
(22) Anmeldetag: 08.06.2017
(51) Int. Cl.: A61F 13/02, A61F 13/00, A61L 15/42

(54) **HAUTAUFLAGE**
DERMAL PATCH
PATCH

(30) Priorität: 08.07.2016 DE 102016008257
(43) Veröffentlichungstag der Anmeldung: 15.05.2019
(73) Patentinhaber: Carl Freudenberg KG, 69469 Weinheim (DE)
(72) Erfinder: HOFBAUER, Thomas, 69493 Hirschberg (DE); BEYER, Daniela, 69469 Weinheim (DE); VILLING-FALUSI, Sandra, 68542 Heddesheim (DE)
(86) Internationale Anmeldenummer: PCT/EP2017/064009
(87) Internationale Veröffentlichungsnummer: WO 2018/007093

(56) Entgegenhaltungen:
- EP-B1- 1 964 580
- DE-A1- 2 631 277
- US-A- 4 538 603
- US-A- 4 728 642
- Anonymous: "Medisponge SuperSoft(TM) Foams", , 3. Dezember 2015 (2015-12-03), XP055388697, Gefunden im Internet: URL:https://web.archive.org/web/2015120320 2550/http://www.essentraporoustechnologies .com/products-and-applications/advanced-wo und-care/medisponge-supersoft-foams [gefunden am 2017-07-06]

## Beschreibung

Die Erfindung betrifft eine Hautauflage, insbesondere eine Wundauflage, umfassend eine Substratlage und eine darauf angeordnete adhäsive Schicht sowie ein Verfahren zu ihrer Herstellung.

### Technisches Gebiet

Kosmetische und medizinische Hautauflagen zur Anwendung am Menschen sind seit langem bekannt.

Eine besonders wichtige Gruppe der Hautauflagen wird in der Wundversorgung eingesetzt.

In der modernen Wundversorgung werden häufig Wundauflagen eingesetzt, die eine Substratlage und eine darauf angeordnete adhäsive Schicht aufweisen. Als Substratlage haben sich insbesondere Polyurethanschäume bewährt, da sie gute klimaregulierende und absorbierende Eigenschaften aufweisen und außerdem im direkten Kontakt zur exsudierenden Wunde platziert sein können. Zudem weisen in der Wundversorgung verwendete Polyurethanschäume in der Regel eine offenzellige Struktur auf, was ihnen eine große Absorptionskapazität verleiht und eine schnelle Aufnahme des Wundexsudats ermöglicht.

Substratlagen werden seit den letzten zwei Jahrzehnten oftmals wundseitig zusätzlich mit einer adhäsiven Schicht versehen, welche auf gesunder Haut haftet, jedoch nicht im Wundkontakt. Im Wundkontakt hat diese, in den meisten Fällen auf Silikon basierende, adhäsive Schicht sogar den Vorteil, dass sie bei feuchten Wunden ein Anhaften bzw. Einwachsen des neu entstandenen Gewebes verhindert, sowie beim Entfernen des Verbands keinen Schmerz verursacht und das erneute Aufreißen der Wunde verhindert.

Nachteilig an der Verwendung von herkömmlichen, auf Silikon basierten adhäsiven Schichten ist, dass sie aufgrund ihrer hydrophoben Eigenschaften und ihrer geringen Wasserdampfdurchlässigkeit eine Aufnahme des Wundexsudats erschweren. Um dies zu kompensieren weist die Silikonadhäsiv-Schicht in der Regel Perforationen auf. Hierdurch soll ermöglicht werden, dass das anfallende Exsudat durch die Klebeschicht in den absorbierenden Schaum gelangen und somit ein ideal feuchtes Wundklima eingestellt werden kann. Trotz der Perforationen ist die Silikonadhäsivschicht zusammenhängend. Dies hat den Nachteil, dass bei Aufnahme von Wundexsudat das freie Quellen der Schaumlage durch die Silikonadhäsivschicht verhindert wird. Hierdurch wölbt sich die Hautauflage von der Wunde weg, wodurch im Folgenden die Aufnahme von weiterem Wundexsudat erschwert wird.

Zum Aufbringen der adhäsiven Schicht hat sich das Transfer-Beschichtungsverfahren etabliert. Dabei wird zuerst eine Folie, z.B. ein Polyurethanfilm, auf einer Seite mit einem Acrylatklebstoff und auf der anderen Seite mit einem weichen Silikon-Adhäsiv, mittels Rakel- und Walzenaufträgen beschichtet. Die dabei verwendeten Silikonadhäsiv-Vorläuferzusammensetzungen bestehen aus zwei Komponenten, die nach dem Vermischen thermisch zu einem weichen, gelartigen Feststoff vernetzen. Das beim Transfer-Beschichtungsverfahren entstehende Sandwich aus Acrylatadhäsiv/Folie/Silikonadhäsiv wird anschließend perforiert und darauf folgend mit der Acrylatklebeseite an das Substrat geklebt.

Ein solches Transfer-Beschichtungsverfahren wird beispielsweise in der EP 2001424 B1 beschrieben. Dabei wird eine Wundauflage hergestellt, die eine Hautkontaktschicht in Form eines ablösbaren Haftlaminats umfasst, das eine Strukturschicht umfasst, die auf wenigstens einem Teil von einer Seite davon ein hydrophobes Gel und auf wenigstens einem Teil der anderen Seite davon einen Haftkleber trägt. Die Strukturschicht besteht vorzugsweise aus einer Kunststofffolie. Nachteilig an der Verwendung dieser Kunststofffolie ist, dass sie eine hohe Steifigkeit und geringe Anpassungsfähigkeit aufweist. In Konsequenz muss zum Ausgleich vergleichsweise viel Silikon verwendet werden, was zu höheren Kosten führt. Außerdem benötigt eine hohe Schichtdicke eine vergleichsweise hohe Energiemenge zum Aushärten der Vorläuferverbindungen. Außerdem verschlechtert die Verwendung einer Folie und einer hohen Menge Silikon die Wasserdampfdurchlässigkeit der Wundauflage.

Die EP 0855921 B1 beschreibt eine Schaumwundauflage und ihre Herstellung. Hierbei wird ein Film mit einem Silikonadhäsiv beschichtet und anschließend ein Polyurethanschaum direkt auf das noch nicht ausgehärtete Silikon aufgelegt. Dieses Laminat wird anschließend durch Aushärtung des Silikons in einem Ofen verbunden. Als Schaummaterial wird ein offenzelliger absorbierender Schaum eingesetzt mit Porengrößen zwischen 30 und 1000 µm. Nachteilig an der beschriebenen Polyurethanschaumstruktur ist, dass aufgrund der Aufnahme des Gels vom Schaum, der aufgenommene Teil des Gels für die Adhäsion zur Haut nicht mehr zur Verfügung steht.

Die US 2004/0138605 A1 beschreibt Wundauflagen mit einem absorbierenden Kern, vorzugsweise aus offenzelligem Schaum, auf dem ein Haftklebstoff angeordnet sein kann. Dieser Haftklebstoff ist bevorzugterweise ein für die Wundversorgung geeignetes Silikon- oder Acrylatadhäsiv. Die Adhäsivlage kann ein Silikon sein, welches auf die (wundseitige) Deckschicht der Wundauflage gesprüht wird.

Auch hier wird aufgrund der offenzelligen Oberfläche eine vergleichsweise große Menge an Silikon benötigt.

Die EP 2696824 B1 beschreibt einen diskontinuierlichen Auftrag eines SilikonAdhäsivs in einem modifizierten Siebdruck- oder Napfwalzenprozess. Als Substrate werden u.a. Polyurethanschäume und Vliesstoffe genannt. Bei diesem Prozess wird ein regelmäßiges Muster aus diskreten kleinen Klebeflächen, z.B. ein Punktmuster, aus Silikonadhäsiv erzeugt. Die Schichtdicke des Silikonadhäsivs beim Siebdruck wird implizit durch die Dicke des Siebs vorgegeben. Diese beträgt 100-2000 µm, sodass die Schichtdicke auch hier vergleichsweise hoch ist.

Die EP 1964580 B1 beschreibt ein Verfahren zur Herstellung einer hydrophilen Polyurethanschaumstruktur, welche ein Silbersalz enthält. Hierbei wird eine schaumbildende Polyurethandispersion bereitgestellt und auf ein Gießpapier aufgebracht. Anschließend wird der Polyurethanschaum ausgehärtet, das Gießpapier entfernt und der Schaum getrocknet. Auf die Oberfläche der so gebildeten, offenzelligen Schaumstruktur wird ein Silikongel aufgebracht und ausgehärtet. Dabei wird ein größerer Teil des Gels vom Schaum aufgenommen.
Nachteilig an der beschriebenen Polyurethanschaumstruktur ist, dass aufgrund der Aufnahme des Gels vom Schaum, der aufgenommene Teil des Gels für die Adhäsion zur Haut nicht mehr zur Verfügung steht.

Wie oben erläutert verteuert der Auftrag einer hohen Menge an Silikon die Produkte in erheblichem Maße. Deswegen ist die Aufgabe der Erfindung eine Hautauflage der eingangs genannten Art bereitzustellen, die es ermöglicht die Dicke der adhäsiven Schicht gering zu halten und eine hohe Performance, insbesondere eine gute Absorptionsfähigkeit für Wundexsudat zu erzielen. Außerdem soll die Hautauflage verrutschsicher, leicht zu entfernen und repositionierbar sein.

### Darstellung der Erfindung

Diese Aufgabe wird gelöst durch eine Hautauflage, insbesondere eine Wundauflage, umfassend eine offenzellige Schaumlage und eine darauf angeordnete, für den Hautkontakt vorgesehene, adhäsive Schicht, wobei zumindest die der adhäsiven Schicht zugewandte Seite der Schaumlage Makroporen aufweist, deren Kavitäten zumindest anteilig durch eine aus der Schaumlage gebildete Sperrschicht überspannt sind dadurch gekennzeichnet, dass der Flächenanteil der Sperrschicht an der Oberfläche der Schaumlage mindestens 20 % beträgt und/oder dass mindestens 20 % der an die Oberfläche der Schaumlage angrenzenden Makroporen durch die Sperrschicht überspannt sind.

Erfindungsgemäß wurde gefunden, dass es durch den Einsatz einer Schaumlage, welche zumindest auf der der adhäsiven Schicht zugewandten Seite eine aus der Schaumlage gebildete Sperrschicht aufweist, möglich ist, nur eine geringe Menge an Adhäsiv zu verwenden und dennoch eine gute Adhäsion zu erreichen. Es wird vermutet, dass das Vorhandensein der Sperrschicht ein Eindringen des Adhäsivs in die Schaumlage zumindest teilweise verhindert. Hierdurch wird weniger Adhäsiv benötigt, um eine lokal geschlossene Beschichtung zu erreichen. Dies ist ein großer Vorteil gegenüber den im Stand der Technik verwendeten, an der Oberfläche offenzelligen Schäumen bei denen eine große Menge an Adhäsiv eingesetzt werden muss. Darüber hinaus bietet das Vorsehen einer aus der Schaumlage gebildeten Sperrschicht den Vorteil, dass keine weiteren Schichten, beispielsweise separate Folien, aufwendig hergestellt, aufgebracht und verklebt werden müssen, die das Eindringen von Adhäsiv verhindern.

Darüber hinaus weist die Hautauflage beim Einsatz als Wundauflage gegenüber auch an der Oberfläche offenzelligen Schäumen den Vorteil auf, dass die Sperrschicht ein Einwachsen von sich neu bildendem Gewebe verhindern kann. Hierdurch kann beim Wechsel der Wundauflage vermieden werden, dass das neu entstandene Gewebe verletzt wird.

Ferner wurde überraschenderweise gefunden, dass die Schaumlage trotz der vorhandenen Sperrschicht eine sehr gute Absorptionsfähigkeit für Wundexsudat, Schweiß und Wasserdampf zeigen kann. So weist die Hautauflage vorteilhafterweise eine Absorptionskapazität von mindestens 5 g/g, beispielsweise von 5 g/g bis 50 g/g, vorzugsweise von 10 g/g bis 30 g/g und noch bevorzugter von 15 g/g bis 25 g/g auf. Ferner ist die Sperrschicht vorzugsweise so ausgebildet, dass die Hautauflage auch eine sehr gute Absorptionszeit aufweist, wobei diese naturgemäß von der Art, Menge und Belegungsgrad der adhäsiven Schicht abhängt.

Erfindungsgemäß weist zumindest die der adhäsiven Schicht zugewandte Seite der Schaumlage, vorzugsweise an die Oberfläche angrenzende, Makroporen auf, deren Kavitäten zumindest anteilig durch eine aus der Schaumlage gebildete Sperrschicht überspannt sind. Diese Ausführungsform ermöglicht es, die Sperrschicht planar auszubilden, wodurch die Adhäsivmenge noch geringer gehalten werden kann. Erfindungsgemäß wird der Begriff "planar" im herkömmlichen Sinne verstanden. Vorzugsweise weist die Sperrschicht eine Planarität, gemessen wie im Abschnitt Messmethoden beschrieben, von weniger als 50 µm, beispielsweise von 0,1 µm bis 50 µm, bevorzugt von 1 µm bis 30 µm, noch bevorzugter von 1 µm bis 20 µm und insbesondere von 1 µm bis 10 µm auf. Der Begriff "Planarität" kann erfindungsgemäß kleinere Unregelmäßigkeiten der Sperrschicht, beispielsweise kleinere Wellen und/oder Faltungen, umfassen.

Unter Makroporen werden erfindungsgemäß Poren verstanden, die einen Porendurchmesser von größer als 25 µm, beispielsweise zwischen 25 µm und 2000 µm und bevorzugt zwischen 100 µm und 500 µm aufweisen.

Erfindungsgemäß weist die Schaumlage zumindest an der der adhäsiven Schicht zugewandten Seite eine Sperrschicht auf, sodass die an die Oberfläche angrenzenden Makroporen zumindest in Richtung zur adhäsiven Schicht zumindest anteilig als geschlossenzellig angesehen werden können. Nichtsdestotrotz ist die Schaumlage im Volumen offenzellig, das heißt, dass die Zellwände im Inneren des Schaums zumindest teilweise zerstört sind. Vorteilhaft verglichen mit einem auch im Volumen geschlossenzelligen Schaum ist beispielsweise im Hinblick auf die Verwendung in einer Wundauflage die höhere Absorptionskapazität für Wundexsudat. Zu diesem Zweck ist die Schaumlage vorteilhafterweise so offenzellig, dass die Schaumlage eine Absorptionskapazität von mindestens 5 g/g, beispielsweise von 5 g/g bis 50 g/g, vorzugsweise von 10 g/g bis 30 g/g und noch bevorzugter von 15 g/g bis 25 g/g aufweist.

Erfindungsgemäß ist die Sperrschicht aus der Schaumlage gebildet, so dass die Sperrschicht als Teil der Schaumlage angesehen und zumindest in manchen Bereichen die Oberfläche der Schaumlage darstellen kann.

Sperrschicht und Schaumlage werden in der Regel materialeinheitlich vorliegen, wobei herstellungsbedingte Anreicherungen einzelner Komponenten als von dem Begriff "materialeinheitlich" umfasst angesehen sein sollen. Erfindungsgemäß können beide oder nur eine Seite der Schaumlage eine aus der Schaumlage gebildete Sperrschicht aufweisen. Wenn eine Seite der Schaumlage die Sperrschicht zu einem größeren Flächenanteil aufweist, so bildet diese Seite vorzugsweise die der adhäsiven Schicht zugewandte Seite.

Zweckmäßigerweise wird die Sperrschicht bei der Herstellung der Schaumlage ausgebildet. Dies kann auf einfache Weise dadurch geschehen, dass eine geeignete Schaummischung frei gegen Luft geschäumt wird. Dabei kann eine Haut an der Oberfläche des Schaums entstehen, welche die Sperrschicht darstellt. Alternativ kann die Ausbildung der Sperrschicht auch durch Aufbringen der Schaummischung auf ein Trägermaterial, beispielsweise ein Gießpapier, erfolgen. Ohne sich auf einen Mechanismus festzulegen wird vermutet, dass beim Kontakt mit dem Gießpapier die an der dem Gießpapier zugewandten Oberfläche angeordneten Makroporen mit der Sperrschicht überspannt ausgebildet werden. Es wurde in praktischen Versuchen gefunden, dass durch die Verwendung von hydrophoben Gießpapieren ein hoher Anteil überspannter Makroporen erzielt werden kann.

Die Sperrschicht kann Mikroporen, d.h. Poren mit einem Porendurchmesser von 25 µm oder weniger, beispielsweise von 0,01 µm bis 25 µm aufweisen. Vorteilhaft an den Mikroporen ist, dass sie der Hautauflage eine verbesserte Durchlässigkeit für Wundexsudat, Schweiß und Wasserdampf verleihen können. Zu diesem Zweck sind die Mikroporen vorzugsweise offenzellig ausgestaltet.

Erfindungsgemäß bevorzugt sind mindestens 20 %, beispielsweise 20 % bis 100 %, vorzugsweise 70 % bis 100 %, noch bevorzugter 90 % bis 100 %, noch bevorzugter 95 % bis 100 % und insbesondere 98 % bis 100 % der an die Oberfläche der Schaumlage angrenzenden Makroporen durch die Sperrschicht überspannt.

In einer weiteren bevorzugten Ausführungsform beträgt der Flächenanteil der Sperrschicht an der Oberfläche der Schaumlage mindestens 20 %, beispielsweise 20 % bis 100 %, vorzugsweise 70 % bis 100 % und insbesondere 95 % bis 100 %.

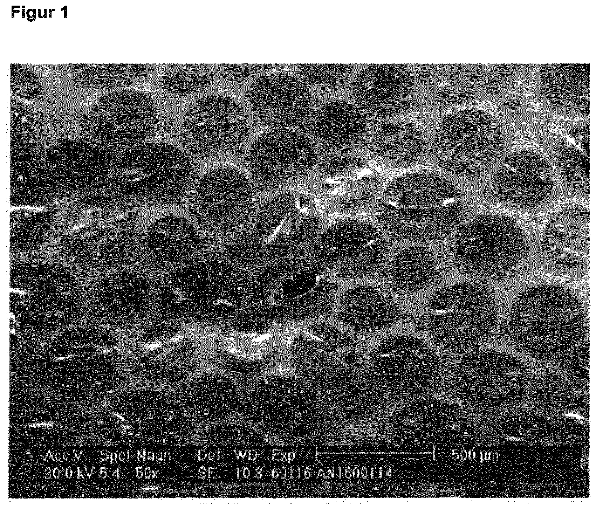

Erfindungsgemäß bevorzugt ist die Sperrschicht dünn. Vorzugsweise liegt ihre Dicke bei weniger als 100 µm, beispielsweise von 0,01 µm bis 100 µm, noch bevorzugter von 0,1 µm bis 50 µm, noch bevorzugter von 0,3 µm bis 20 µm, noch bevorzugter von 0,4 µm bis 10 µm und insbesondere von 0,5 µm bis 3 µm. In einer bevorzugten Ausführungsform ist die Sperrschicht so dünn, dass in Aufsicht bei einer optischen Auswertung von REM-Bildern die Makroporen bei einer Vergrößerung von beispielsweise 30 fach unterhalb der Sperrschicht noch zu erkennen sind, wie beispielsweise in Figur 1. In Figur 1 wird eine unbeschichtete Schaumlage gezeigt. Ist die adhäsive Schicht ausreichend dünn, so kann auch eine beschichtete Schaumlage auf die gleiche Weise untersucht werden. Vorteilhaft an einer dünnen Sperrschicht ist, dass sie eine höhere Permeabilität für Wundexsudat, Schweiß und Wasserdampf aufweist und dennoch für in der Wundversorgung übliche Adhäsive, insbesondere für Silikonadhäsive und deren Vorläuferzusammensetzungen, undurchlässig ist.

Erfindungsgemäß bevorzugt werden die Kavitäten der an die Oberfläche der Schaumlage angrenzenden Makroporen zumindest anteilig durch die Sperrschicht überspannt. Dabei ist unter dem Begriff Überspannen ein Bedecken der Kavitäten unter Bildung eines geschlossenen Hohlraums zu verstehen. Funktional soll durch das Überspannen mit der Sperrschicht ein Eindringen des flüssigen Adhäsivs oder seiner Vorläuferzusammensetzungen zumindest teilweise verhindert werden. Bei einer Perforation der Sperrschicht kann das Eindringen des Adhäsivs oder seiner Vorläuferzusammensetzungen zumindest an dieser Stelle nicht mehr zuverlässig verhindert werden.

Durch die Barrierefunktion der Sperrschicht ist es möglich, die adhäsive Schicht unmittelbar auf die Oberfläche der Schaumlage aufzubringen, so dass ein Eindringen des Adhäsivs in die Schaumlage verhindert oder zumindest verringert werden kann. In einer bevorzugten Ausführungsform der Erfindung ist mithin die adhäsive Schicht unmittelbar auf der Oberfläche der Schaumlage angeordnet, wobei unmittelbar so zu verstehen ist, dass keine weiteren Lagen zwischen adhäsiver Schicht und Schaumlage angeordnet sind.

In einer bevorzugten Ausführungsform der Erfindung weist die adhäsive Schicht eine Dicke von weniger als 300 µm, beispielsweise zwischen 1 µm und 300 µm, vorzugsweise zwischen 1 µm und 100 µm, noch bevorzugter zwischen 1 µm und 90 µm, noch bevorzugter zwischen 1 µm und 50 µm, noch bevorzugter zwischen 2 µm und 30 µm, noch bevorzugter zwischen 3 µm und 20 µm und insbesondere zwischen 5 µm und 15 µm auf.

Vorteilhaft an einer dünnen adhäsiven Schicht ist, dass die Wasserdampfdurchlässigkeit höher ist und gegebenenfalls in der Schaumlage enthaltene aktive Substanzen in geringerer Entfernung zu ihrem Wirkort in der Wunde vorliegen können.

In einer weiteren bevorzugten Ausführungsform der Erfindung beträgt die Auftragsmenge der adhäsiven Schicht weniger als 200 g/m², beispielsweise von 1 g/m² bis 200 g/m², vorzugsweise von 1 g/m² bis 100 g/m², noch bevorzugter von 1 g/m² bis 90 g/m², noch bevorzugter von 1 g/m² bis 50 g/m², noch bevorzugter 2 g/m² bis 30 g/m², noch bevorzugter 3 g/m² bis 20 g/m² und insbesondere von 5 g/m² bis 15 g/m².

Vorteilhaft an einer geringen Auftragsmenge der adhäsiven Schicht ist die Reduktion der anfallenden Kosten, insbesondere der Material- und Energiekosten.

In praktischen Versuchen wurde festgestellt, dass die erfindungsgemäße Hautauflage auch bei geringer Auftragsmenge der adhäsiven Schicht eine gute Adhäsion zeigen kann. Somit beträgt die Adhäsion der Hautauflage gegen ein Stahlsubstrat auch bei den vorgenannten geringen Auftragsmengen, d.h. beispielsweise bei weniger als 200 g/m², vorzugsweise mehr als 0,05 N/2,5 cm, beispielsweise von 0,05 N/2,5 cm bis 5 N/2,5 cm, vorzugsweise von 0,05 N/2,5 cm bis 1 N/2,5 cm und besonders bevorzugt von 0,05 N/2,5 cm bis 0,2 N/2,5 cm.

Die adhäsive Schicht kann die Schaumlage teilweise oder vollflächig bedecken. Erfindungsgemäß bevorzugt bedeckt die adhäsive Schicht die Schaumlage nur teilweise, da hierdurch die Permeation von Wundexsudat erleichtert wird. In diesem Fall beträgt der Belegungsgrad vorzugsweise weniger als 99 %, beispielsweise von 10 % bis 99 %, vorzugsweise von 30 % bis 95 % und insbesondere von 50 % bis 90 %.

Eine teilweise Bedeckung der Schaumlage kann auf verschiedene Arten und Weisen erfolgen, beispielsweise durch Schablonenauftrag oder durch nachträgliches, bereichsweises Entfernen der adhäsiven Schicht. Dieses Entfernen kann dabei so erfolgen, dass zusätzlich auch Anteile der darunter liegenden Schaumlage, inklusive der Sperrschicht, mit entfernt werden. An diesen Stellen wird die Oberfläche der Schaumlage folglich nicht durch die Sperrschicht gebildet.

In dieser Ausgestaltung der Erfindung werden vorzugsweise von 10 % bis 99 %, noch bevorzugter von 30 % bis 95 % und insbesondere von 70 % bis 90 % der Oberfläche der Schaumlage von der Sperrschicht gebildet.

Diejenigen Bereiche der Schaumlage, die keine Sperrschicht mehr aufweisen, sind auch nicht mit der adhäsiven Schicht bedeckt und beispielsweise als Vertiefungen und/oder Löcher ausgestaltet. Die Vertiefungen und/oder Löcher können die verschiedensten Formen eines regulären oder irregulären Musters, beispielsweise als Gitter-, Loch-, Punkt-, Strich-, Linien-, Vieleck-, beispielsweise Rauten- oder Waben-, Kreis-, Kreuz-, Flecken und/oder Insel-Muster, annehmen. Von den in der Schaumlage vorhandenen Mikroporen bzw. Makroporen können die Vertiefungen und/oder Löcher beispielsweise dadurch unterschieden werden, dass sie nachträglich eingebracht wurden.

Vorteilhaft an dem Vorhandensein der Vertiefungen und/oder Löcher ist, dass an diesen Stellen das Wundexsudat besonders gut eindringen kann, wodurch die Absorptionszeit gezielt verringert werden kann.

In einer besonders bevorzugten Ausführungsform der Erfindung weist die Schaumlage eine nur teilweise Bedeckung mit der adhäsiven Schicht auf. Bei einer teilweisen Bedeckung kann die adhäsive Schicht in Form eines regulären oder irregulären Musters, beispielsweise als Gitter-, Loch-, Punkt-, Strich-, Linien-, Vieleck-, beispielsweise Rauten- oder Waben-, Kreis-, Kreuz-, Flecken und/oder Insel-Muster, vorliegen.

In einer bevorzugten Ausführungsform liegt die adhäsive Schicht als unzusammenhängendes Muster, beispielsweise in Form eines inselartigen Musters, vor. Vorteilhaft an dieser Ausführungsform ist, dass das freie Quellen der Schaumlage bei Aufnahme von Wundexsudat in Richtung Wundbett ermöglicht wird und damit weiterhin Exsudataufnahme gewährleistet werden kann.

Als besonders geeignete Muster haben sich ferner ineinander greifende Muster erwiesen. Vorteilhaft an ineinander greifenden Mustern ist, dass sie eine unerwünschte, laterale Ausbreitung des Wundexsudats verhindern und somit einer Mazeration des Wundrands vorbeugen.

Die adhäsive Schicht kann unterschiedliche Materialien enthalten. Bevorzugt sind Materialien oder Materialkombinationen, welche auf gesunder Haut haften, jedoch nicht im Wundkontakt. So werden die Materialien für die adhäsive Schicht vorteilhafterweise so ausgewählt, dass bei feuchten Wunden ein Anhaften bzw. Einwachsen neu entstehenden Gewebes verhindert, beim Entfernen des Verbands kein Schmerz verursacht und/oder das erneute Aufreißen der Wunde verhindert wird.

Zur Verwendung in der adhäsiven Schicht geeignete Materialien oder Materialkombinationen sind adhäsive Materialien, die im Hinblick auf den Einsatz als Hautauflage eine ausreichend haftende und ausreichend verrutschsichere Wirkung zeigen. Besonders bevorzugt sind Materialien, die zumindest für einen Zeitraum, der ausreicht um eine sekundäre Befestigung, beispielsweise einen Verband, anzubringen, eine ausreichend haftende und ausreichend verrutschsichere Wirkung zeigen. Besonders geeignet sind Silikone, insbesondere Silikongele, Silikonelastomere und/oder mit Substituenten wie Polyethylenglykol und/oder Polyurethan modifizierte quervernetzte Polyorganosiloxane. Weiterhin geeignet sind Polyurethane, wie etwa Polyurethangele oder Polyurethanelastomere. Weiterhin geeignet sind teilweise oder vollständig gehärtete hydrophile oder hydrophobe Gele, beispielsweise, Hydrogele, insbesondere auf Acrylat- und/oder Monosacharid-Basis, sowie Gemische und/oder Copolymere hiervon, Hydrokolloidmassen und/oder salbenartige Gemische aus festen und flüssigen Kohlenwasserstoffen, insbesondere Vaseline.

In einer erfindungsgemäß besonders bevorzugten Ausführungsform enthält die adhäsive Schicht Silikone. Erfindungsgemäß können die verschiedensten Silikone, insbesondere Silikongele, eingesetzt werden, sofern sie die gewünschten adhäsiven Eigenschaften aufweisen.

Silikone sind anorganisch/organische Polymere basierend auf sich wiederholenden -Si-O-Einheiten mit organischen Seitenketten. Silikone können über Siloxanbrücken Netzwerke bilden und/oder die organischen Seitenketten können ihrerseits über kovalente Bindungen Netzwerke ausbilden. Die erfindungsgemäß eingesetzten Silikone sollen, wie oben erläutert, adhäsive Eigenschaften zeigen. Der Fachmann weiß, wie die Silikone auszuwählen sind, damit sie die gewünschten, adhäsiven Eigenschaften zeigen, beispielsweise durch gezielte Einstellung des Vernetzungs- und/oder Verschlaufungsgrades sowie der Netzwerkstruktur und/oder -dichte.

Erfindungsgemäß bevorzugte Silikone sind Polyorganosiloxane, insbesondere Polyorganosiloxane der allgemeinen Formel I 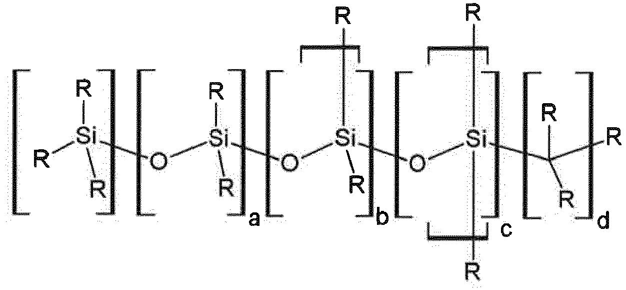 wobei die Reste R unabhängig voneinander ausgewählt sind aus der Gruppe bestehend aus Wasserstoff C1-C8-Alkyl-, C6-C14-Aryl-, C4-C18-Arylalkyl-, C4-C18-Alkylaryl, C2-C8-Alkenyl, C1-C8-Alkyliden-, C1-C8-Alkoxy(C1-C8)-alkyl- oder Poly(alkylenoxy)alkylgruppen. Die vorgenannten Gruppen können linear oder verzweigt vorliegen sowie substituiert oder unsubstituiert, wobei a, b, c und d unabhängig voneinander eine Zahl zwischen 0 und 10000, vorzugsweise von 1 bis 5000 sind.

Vorzugsweise sind die Reste R unabhängig voneinander ausgewählt aus der Gruppe bestehend aus Wasserstoff, C1-C8-Alkylreste, wie der Methyl-, Ethyl-, n-Propyl-, Isopropyl-, n-Butyl-, sec.-Butyl-, Amyl-, Hexylrest; C2-C8-Alkenylreste, wie der Vinyl-, Allyl-, und Butenylrest; C6-C14-Arylreste, wie der Phenylrest; C1-C8-Alkylidenreste wie der Methylen-, Ethylen-, Propylen- und Butylenrest.

Besonders bevorzugt sind die Reste R unabhängig voneinander ausgewählt aus der Gruppe bestehend aus Wasserstoff, Methyl, Ethyl, Propyl, Phenyl, Vinyl, Ethylen.

In Formel I sind die nachfolgenden Einheiten 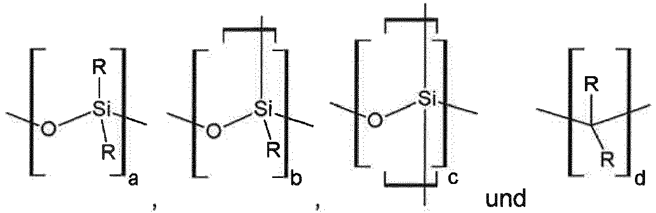 repetitive Einheiten. Diese können, wie dem Fachmann bekannt ist, im Polymer in unterschiedlicher Abfolge, das heißt beispielsweise blockweise, statistisch verteilt und/oder wiederkehrend vorliegen.

Die Einheit 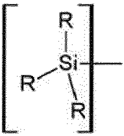 und die Reste -R in Formel I sind Endgruppen.

Bevorzugt sind die erfindungsgemäß geeigneten Silikone additionsvernetzende Silikonzusammensetzungen. Hierbei kann es sich um Einkomponenten-Silikonzusammensetzungen wie auch um Zwei- oder Mehr-Komponenten-Silikonzusammensetzungen, bevorzugt um Zweikomponenten-Silikonzusammensetzungen (Komponente A und B), handeln. Dabei sind raumtemperaturvernetzende Zweikomponenten-Silikonzusammensetzungen ganz besonders bevorzugt. Unter raumtemperaturvernetzend wird eine Vernetzung bei Temperaturen ab 15 °C verstanden.

Zur Herstellung der Silikone ausgehend von den Zweikomponenten-Silikonzusammensetzungen wird bevorzugt ein geeigneter Katalysator, bevorzugt ein Schwermetall und insbesondere ein Platin-Katalysator eingesetzt. Als geeignet haben sich insbesondere flüssige Zwei-Komponenten-Systeme aus Silikonvorläuferverbindungen erwiesen, bei welchen eine Komponente den Katalysator enthält. Die Silikonvorläuferverbindungen können vermischt werden und auf ein Substrat gegeben werden, wo sie zu dem Silikongel aushärten.

Besonders bevorzugt enthält die Komponente (A) vinylfunktionelle, im Wesentlichen lineare oder geringfügig verzweigte Polydiorganosiloxane mit einer Viskosität von 10 bis 100000 mPa·s, bevorzugt von 100 bis 10000 mPa·s, besonders bevorzugt von 200 bis 2000 mPa·s jeweils bei 25 °C.

Besonders bevorzugt enthält die Komponente (B) Si-H-funktionelle, im Wesentlichen lineare oder geringfügig verzweigte Polydiorganosiloxane mit einer Viskosität von 10 bis 100000 mPa·s, bevorzugt von 100 bis 10000 mPa·s, besonders bevorzugt von 200 bis 2000 mPa·s jeweils bei 25 °C.

Zusätzlich enthält die Komponente A und/oder B, vorzugsweise die Komponente A, den Katalysator.

In einer besonders bevorzugten Ausführungsform liegt das Silikon in Form eines Silikongels vor. Unter einem Silikongel wird erfindungsgemäß ein Silikon verstanden, welches ein Polymernetzwerk suspendiert in einer flüssigen Solphase aufweist. Bevorzugt ist das Silikongel viskoelastisch und/oder weist eine Penetration von 20 bis 500 1/10 mm, noch bevorzugter von 50 bis 300 1/10 mm auf (gemessen in Anlehnung an DIN ISO 2137). Vorteilhaft an der Verwendung von Silikongelen ist, dass sie gleichzeitig bei guter Haftung auf der Haut auch eine atraumatische Entfernung ermöglichen.

Für die erfindungsgemäßen Zwecke geeignete Zweikomponenten-Silikonzusammensetzungen sind aus dem Stand der Technik bekannt und zum Beispiel in den EP 0251810 A1, EP 0300620 A1 und US 4921704 A1 beschrieben. Diese Systeme umfassen im Wesentlichen eine ein Vinylsubstituiertes Polydiorganosiloxan, insbesondere Polydimethylsiloxan, und einen Platin-Katalysator enthaltende Komponente A. Die Komponente B beinhaltet das Polydiorganosiloxan das an das Siliziumatom direkt gebundene Wasserstoffatome aufweist. Gegebenenfalls können die Systeme weitere Additive wie Füllstoffe, therapeutisch wirksame Substanzen, wie etwa Silberionen, Pigmente, Stabilisatoren und/oder Inhibitoren enthalten.

Durch das Zusammenfügen der beiden Komponenten kann die metallkatalysierte Additionsreaktion der Vinyl- und Si-H-Gruppen, die zur Vernetzung und Aushärtung der Polydiorganosiloxane führt, gestartet werden. Hierbei kann das Silikon in Schichtform entstehen und zu einem vernetzten Gel aushärten. Die Eigenschaften des ausgehärteten Silikongels können dabei auf unterschiedliche Weise beeinflusst werden, beispielsweise durch eine Variation des Verhältnisses der Komponenten A und B, durch Variation der Molekulargewichte und/oder des Verzweigungsgrades der eingesetzten Polysiloxane, durch die Variation der Gehalte der für die Quervernetzung verantwortlichen Gruppen in den Komponenten A und B sowie ihrer Verteilung auf den Polydiorganosiloxanvorläufermolekülen (Funktionalität) oder durch die Konzentration des/der gegebenenfalls zugefügten Additive. So können Silikongele erzeugt werden, die angenehm weich im Griff sind, eine hohe Kohäsion und gleichzeitig eine signifikante Adhäsion zur Haut aufweisen.

Silikongel-Vorläuferzusammensetzungen sind beispielsweise kommerziell von der Firma Wacker unter der Produktbezeichnung Silpuran® und den Typennummern 2110, 2112, 2120 oder 2130, oder von der Firma NuSil Technologies unter den Produktbezeichnungen MED-6342, MED-6345 oder MED-6350 oder von der Firma Dow Corning GmbH unter den Produktbezeichnungen MG 7-9800, MG 7-9850 oder MG 7-9900 erhältlich.

Vorzugsweise enthält die adhäsive Schicht die adhäsiven Materialien, beispielsweise die vorgenannten Materialien, und insbesondere die Silikone, in einer Menge von mindestens 20 Gew%, beispielsweise von 20 Gew% bis 100 Gew% und noch bevorzugter von 50 Gew% bis 100 Gew% und insbesondere von 80 Gew% bis 100 Gew%.

Erfindungsgemäß weist die Hautauflage eine Schaumlage auf. Vorteilhaft an der Verwendung der Schaumlage als Wundauflage ist, dass sie überschüssiges Wundexsudat aufnehmen kann und damit einhergehend ein optimales Klima zur Verbesserung der Wundheilung einstellen kann. Außerdem kann sie eine Ausfüllung des Wundbetts durch ihr Anschwellen bei Aufnahme des Wundexsudats ermöglichen. Dies ist vorteilhaft, weil es eine Aufnahme von Wundexsudat über die gesamte Tragedauer ermöglichen kann.

Die Schaumlage ist erfindungsgemäß dadurch gekennzeichnet, dass zumindest die der adhäsiven Schicht zugewandte Seite Makroporen aufweist, die vorzugsweise an die Oberfläche der Schaumlage angrenzen und deren Kavitäten zumindest anteilig durch eine aus der Schaumlage gebildete Sperrschicht überspannt sind. Wie bereits oben erläutert, kann diese spezielle Struktur der Oberfläche der Schaumlage auf einfache Weise dadurch bewirkt werden, dass eine Schaummischung gegen Luft aufgeschäumt wird. Alternativ kann die Schaummischung auf ein Trägermaterial, beispielsweise ein Gießpapier, gegossen werden. Ohne sich auf einen Mechanismus festzulegen wird vermutet, dass beim Kontakt mit dem Gießpapier die an der dem Gießpapier zugewandten Oberfläche angeordneten Makroporen geschlossenzellig ausgebildet werden.

Die Schaumlage weist vorzugsweise einen Polymerschaum auf und besteht noch bevorzugter aus einem solchen. Es kann jeder in der modernen Wundbehandlung übliche Polymerschaum Anwendung finden. Insbesondere können als Polymerschaum ein Polyurethanschaum, ein Polyetherschaum, ein Polyurethan-Polyether-Copolymerschaum, ein Polyvinylacetatschaum, ein Polyvinylalkoholschaum, ein Kollagenschaum, ein Chitosanschaum oder Mischungen dieser Schäume eingesetzt werden. Dabei ist ein Polyurethanschaum besonders bevorzugt. In einer bevorzugten Ausführungsform kann als Polymerschaum ein hydrophiler Polymerschaum eingesetzt werden, wobei die vorgenannten Polymere vorzugsweise hydrophil vorliegen. Ganz besonders bevorzugt ist der Polymerschaum ein hydrophiler Polyurethanschaum. Im Zusammenhang mit der vorliegenden Erfindung ist unter einem hydrophilen Polymerschaum ein solcher Polymerschaum zu verstehen, der Flüssigkeiten absorbieren und/oder speichern kann.

Der hydrophile Polyurethanschaum kann die verschiedensten Präpolymere und Hilfsstoffe enthalten.

Der hydrophile Polyurethanschaum kann durch Mischen eines Isocyanatterminierten Polyethers als Präpolymer mit einer Funktionalität von mehr als zwei mit einem oberflächenaktiven Mittel und Wasser und Gießen der Mischung auf eine Oberfläche hergestellt werden.

Die folgende Darstellung beschreibt schematisch einen bevorzugten Ablauf des Herstellungsverfahrens einer Polyurethan-Schaumlage im Gießverfahren ("cast to thickness"):

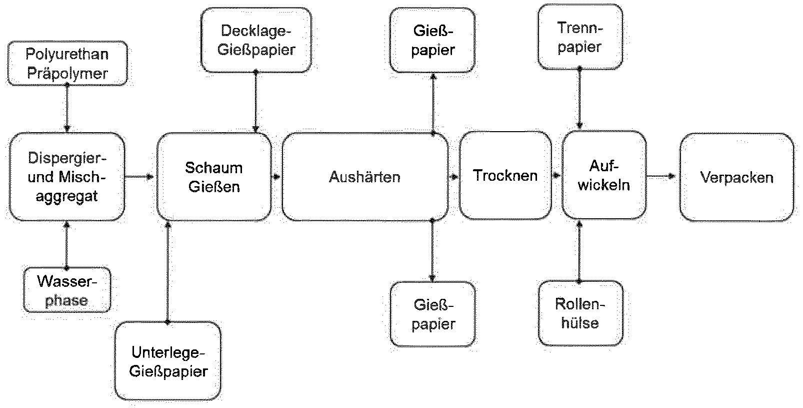

Die Präpolymere können als Reaktionsprodukte aus Polyalkylenglykolethern, ethoxylierten Glycerinen und/oder Polyalkylenglykolmonoalkarylethern mit aliphatischen und/oder aromatischen Diisocyanaten, wie Toluol-2,4-diisocyanat, Methylendi(phenylisocyanat), Hexamethylendiisocyanat und/oder aliphatischen und/oder aromatischen Polyisocyanaten hergestellt werden.

Als Hilfsstoffe können verwendet werden: Detergenzien, besonders bevorzugt nicht-ionische Detergenzien und ganz besonders bevorzugt Detergenzien auf der Basis von Polyethylenglykolmonolaurylethern mit einem mittleren Molekulargewicht von etwa 350 bis 1100, Polyethylenglykolmonomethylethern mit einem mittleren Molekulargewicht zwischen 500 und 5000 und/oder Polyalkylenglykolethern.

Des Weiteren können noch weitere Hilfsstoffe beigesetzt werden, wie zum Beispiel Antioxidanzien, antimikrobielle Wirkstoffe, wie zum Beispiel Silber, Silber enthaltende Substanzen, Polyhexamethylbiguanid, lod, Chlorhexidin, Honig, Essigsäure und/oder Kaliumpermanganat und/oder antibakterielle Substanzen auf der Basis von quartärnisierten Ammoniumsalzen.

In einer weiteren bevorzugten Ausführungsform der Erfindung weist die Schaumlage eine mittlere Dicke von mindestens 0,5 mm, vorzugsweise von 1 mm bis 10 mm und insbesondere von 1 mm bis 7 mm auf.

In einer weiteren bevorzugten Ausführungsform der Erfindung beträgt die mittlere Dichte der Schaumlage von mindestens 50 kg/m³, vorzugsweise von 70 kg/m³ bis 150 kg/m³ und insbesondere von 90 kg/m³ bis 150 kg/m³.

Erfindungsgemäß weist die Hautauflage eine Schaumlage und eine darauf angeordnete adhäsive Schicht auf. Zusätzlich kann die Hautauflage noch weitere Schichten enthalten. So ist es für manche Anwendungen bevorzugt, wenn eine Flüssigkeitsbarrierelage auf der der adhäsiven Schicht abgewandten Seite der Schaumlage angeordnet ist, um einen unerwünschten Flüssigkeitsaustritt aus der Hautauflage zu verhindern. Als Flüssigkeitsbarrierelage können die verschiedensten Schichten, beispielsweise Polyurethanfolien, verwendet werden.

Ebenfalls denkbar ist die Verwendung weiterer, vorzugsweise absorbierender Lagen und/oder Schichten, beispielsweise einer (super)absorbierenden Schicht, wie eines (super)absorbierenden Vliesstoffs und/oder (super)absorbierender Partikel. Diese weiteren absorbierenden Lagen und/oder Schichten sind vorzugsweise auf der der adhäsiven Schicht abgewandten Seite der Schaumlage und gegebenenfalls zwischen der Schaumlage und einer Flüssigkeitsbarrierelage angeordnet.

Ein weiterer Gegenstand der Erfindung umfasst ein Verfahren zur Herstellung einer Hautauflage. Dieses Verfahren umfasst die folgenden Schritte:
(A) Herstellen und/oder Bereitstellen einer Schaumlage, die zumindest auf einer Seite Makroporen aufweist, deren Kavitäten zumindest anteilig durch eine aus der Schaumlage gebildete Sperrschicht überspannt sind so, dass der Flächenanteil der Sperrschicht an der Oberfläche der Schaumlage mindestens 20 % beträgt und/oder dass mindestens 20 % der an die Oberfläche der Schaumlage angrenzenden Makroporen durch die Sperrschicht überspannt sind;
(B)auftragen eines adhäsiven Materials und/oder Vorläuferverbindungen hiervon auf die zumindest eine Seite der Schaumlage, die die Sperrschicht aufweist;
(C)gegebenenfalls Aushärten des adhäsiven Materials und/oder Vorläuferverbindungen hiervon unter Ausbildung einer adhäsiven Schicht.

In Schritt (A) wird eine Schaumlage hergestellt und/oder bereitgestellt, die zumindest auf einer Seite Makroporen aufweist, deren Kavitäten zumindest anteilig durch eine aus der Schaumlage gebildete Sperrschicht überspannt sind. Die Herstellung der Schaumlage kann auf verschiedene, beispielsweise die oben erläuterten, Arten und Weisen erfolgen. Die vorteilhafte Beschaffenheit der Schaumlage kann dabei wie oben erläutert beispielsweise durch Aufschäumen gegen Luft und/oder durch die Verwendung eines hydrophoben Gießpapiers erreicht werden.

In Schritt (B) wird ein adhäsives Material und/oder eine Vorläuferverbindung hiervon auf zumindest eine Seite der Schaumlage aufgetragen. Die erfindungsgemäß besonders geeigneten adhäsiven Materialen und/oder Vorläuferverbindungen hiervon wurden bereits oben genannt.

Das Auftragen des adhäsiven Materials und/oder der Vorläuferverbindungen hiervon kann auf die verschiedensten Arten und Weisen erfolgen. Als besonders geeignet haben sich Sprühen, Walzenauftrag, insbesondere Roll Kiss Coating, und Schlitzdüsenauftrag erwiesen, da sich diese Verfahren besonders gut zum Aufbringen von Beschichtungen mit geringem Flächengewicht eignen ohne die Sperrschicht zu verletzen. Der Auftrag erfolgt bevorzugt durch kontaktfreie oder kontaktarme Auftragsverfahren. So hat sich der Auftrag mittels Sprühen als besonders geeignet herausgestellt.

In den vorgenannten Verfahren hat es sich als zweckmäßig erwiesen, das adhäsive Material und/oder Vorläuferverbindungen hiervon mit einer Viskosität von weniger als 5500 mPa·s, vorzugsweise von 1 mPa·s bis 3000 mPa·s und noch bevorzugter von 100 mPa·s bis 1500 mPa·s aufzubringen.

Soll der Auftrag zu einer nur teilweisen Bedeckung mit der adhäsiven Schicht führen, so hat sich der Auftrag durch eine Schablone (Schablonenauftrag) als besonders geeignet erwiesen. Dabei wird die Schaumlage zweckmäßigerweise vor Auftrag des adhäsiven Materials und/oder der Vorläuferverbindungen hiervon mit einer Schablone bedeckt, wobei die Schablone Öffnungen der gewünschten Muster enthält. Nach dem Auftrag des adhäsiven Materials und/oder der Vorläuferverbindungen hiervon kann die Schablone entfernt werden.

Werden Vorläuferverbindungen des adhäsiven Materials eingesetzt, so werden diese bevorzugt vor dem Auftrag auf die Schaumlage vermischt. In einer weiteren bevorzugten Form erfolgt die Zusammenführung der Vorläuferverbindungen erst auf der Schaumlage.

In Abhängigkeit von den eingesetzten Materialien kann durch die Schritte (A) und (B) direkt eine adhäsive Schicht gebildet werden. In einer bevorzugten Ausführungsform der Erfindung erfolgt nach den Verfahrensschritten (A) und (B) eine Aushärtung des adhäsiven Materials und/oder Vorläuferverbindungen hiervon unter Ausbildung der adhäsiven Schicht (C).

In einer bevorzugten Ausführungsform der Erfindung schließt sich an die Schritte (A), (B) und gegebenenfalls (C) ein weiterer Verfahrensschritt (D) an, in dem Vertiefungen und/oder Perforationen in die adhäsive Schicht und/oder die Schaumlage eingebracht werden. Dies kann beispielsweise durch das lokale Abtragen von Teilen der adhäsiven Schicht und/oder der Schaumlage erfolgen. Hierfür kann beispielsweise ein Laser, insbesondere ein CO₂-Laser, eingesetzt werden.

Die Hautauflage kann für die verschiedensten, medizinischen und nicht-medizinischen Anwendungen eingesetzt werden. Im nicht-medizinischen Bereich sind beispielsweise Anwendungen denkbar, bei denen die Feuchtigkeit in Hautnähe reguliert werden soll, also als feuchtigkeitsregulierende Hautauflage. Im medizinischen Bereich ist die Verwendung zur Wundbehandlung erfindungsgemäß besonders bevorzugt.

Wie oben erläutert ist es aufgrund der vorhandenen Sperrschicht möglich, eine geringe Schichtdicke der adhäsiven Schicht bei dennoch guter Adhäsionseigenschaft zu erzielen. Vorteilhaft an einer dünnen adhäsiven Schicht ist, dass gegebenenfalls in der Schaumlage enthaltene aktive Substanzen in geringerer Entfernung zu ihrem Wirkort in der Wunde vorliegen können und die Wasserdampfdurchlässigkeit verbessert werden kann.

Des Weiteren können durch die geringe Schichtdicke niedrige Schälkräfte erreicht werden, wodurch das Entfernen der Hautauflage hautschonender gestaltet werden kann.

Darüber hinaus weist die Hautauflage beim Einsatz als Wundauflage gegenüber auch an der Oberfläche offenzelligen Schäumen den Vorteil auf, dass die Sperrschicht ein Einwachsen von sich neu bildendem Gewebe verhindern kann. Hierdurch kann beim Wechsel der Wundauflage vermieden werden, dass das neu entstandene Gewebe verletzt wird.

Die erfindungsgemäße Hautauflage weist eine offenzellige Schaumlage auf und kann hierdurch eine gute Absorptionskapazität und Absorptionszeit zeigen. Daher eignet sie sich hervorragend zur Erzeugung eines feuchten Wundklimas und mithin zur feuchten Wundbehandlung, insbesondere von chronischen Wunden.

Darüber hinaus sind auch weitere medizinische Verwendungen denkbar, beispielsweise zur Verhinderung von Druckgeschwüren (Dekubitusprävention).

### Messmethoden

Für die Zwecke der vorliegenden Erfindung wurden folgende Messmethoden angewandt:
Grundsätzlich gilt, dass bei allen Messmethoden, bei denen Mittelwerte gebildet werden, der Fachmann die Anzahl der zur Mittelung bestimmten Werte in Abhängigkeit von deren Streuung wählt. Je größer die gefundenen Abweichungen sind, desto mehr Werte wird er in die Bestimmung einbeziehen.

**Optische Auswertung von REM-Aufnahmen:** Untersuchungen mittels Rasterelektronenmikroskopie werden mit einer Beschleunigungsspannung von 20 kV durchgeführt. Um Aufladungseffekte und daraus resultierende Messfehler zu vermeiden, werden die Proben vor der REM-Untersuchung mit Gold besputtert. Dies geschieht bei einem Argongasdruck von 0,1 mbar bei 30 mA Sputterstrom in einem Abstand von 10 cm. Die Sputterzeit beträgt 300 Sekunden.

**Messung der Planarität der Sperrschicht:** Die Planarität wird durch optische Auswertung von REM-Messungen bestimmt. Es wird eine fiktive Referenzoberfläche dadurch generiert, dass ein beidseitig Polyethylen-beschichtetes Papier mit einem Flächengewicht von 120 g/m² auf die adhäsive Schicht aufgelegt wird. Zur Bestimmung der Planarität wird die Distanz zwischen Unterseite des Papiers und der höchsten, schaumaufweisenden Stelle an mindestens 10 Messstellen gleichmäßig verteilt über einen Bereich von mindestens 2 mm bestimmt. Es wird der gestutzte Mittelwert und die Standardabweichung bestimmt. Der gestutzte Mittelwert wird durch Streichen der 10 % höchsten und 10 % niedrigsten Werte gebildet. Die ermittelte Standardabweichung der gestutzten Stichprobe entspricht der Planarität.

**Dicke der Sperrschicht:** Die Dicke wird durch optische Auswertung von REM-Messungen eines Querschnitts der Sperrschicht bestimmt. Hierbei sind die Bereiche heranzuziehen, an denen Makroporen vorliegen. Es wird über mindestens 5 Werte gemittelt.

**Die Dicke der adhäsiven Schicht:** Es wird eine fiktive Referenzoberfläche dadurch generiert, dass ein beidseitig Polyethylen-beschichtetes Papier mit einem Flächengewicht von 120 g/m² auf die adhäsive Schicht aufgelegt wird. Die Dicke wird als Distanz zwischen Unterseite des Papiers und der tiefsten, adhäsiventhaltenden Stelle an der jeweiligen Messstelle bestimmt. Die Auswertung erfolgt mittels REM im Querschnitt. Falls es den Kontrast zwischen Adhäsiv und Schaumlage erhöht, wird ein Rückstreudetektor verwendet. Die Dicke wird an mindestens 10 Stellen gleichmäßig verteilt über einen Bereich von mindestens 2 mm gemessen und der Mittelwert bestimmt. Um eine Verfälschung durch nachträgliches Eindringen von Adhäsiv in die Schaumlage beim Bilden der Querschnittsfläche zu vermeiden, wird der Schnitt senkrecht von der dem Adhäsiv abgewandten Seite der Schaumlage aus geführt.

**Auftragsmenge der adhäsiven Schicht:** Die Auftragsmenge der adhäsiven Schicht wird durch Auswiegen vor und nach der Beschichtung und durch Differenzbildung ermittelt. Vorzugsweise beträgt die Probengröße mindestens 100cm².

**Belegungsgrad der adhäsiven Schicht:** Der Belegungsgrad wird durch optische Auswertung von REM-Messungen vorzugsweise in Aufsicht aufgenommen an der adhäsiven Schicht ermittelt. Falls es den Kontrast zwischen Adhäsiv und Schaumlage erhöht, wird ein Rückstreudetektor verwendet. Vorzugsweise beträgt die Probengröße mindestens 4cm².

**Bestimmung der Adhäsion:** Durch Zugversuche wird an den beschichteten, 25 mm breiten Proben die Adhäsion bestimmt. Dafür wird in Anlehnung an DIN EN 1939:2003 der Median der Spitzen-Schälwiderstände mit einer Spitzendefinition von 0,5 mN gegen ein gereinigtes Stahlsubstrat bestimmt. Der Abzugswinkel beträgt 180 ° und die Abzugsgeschwindigkeit 300 mm/min. Die Adhäsion wird in der Einheit N/2,5 cm angegeben.

**Porendurchmesser der Poren in der Schaumlage:** Der Porendurchmesser wird durch optische Auswertung von REM-Aufnahmen durch Anlegen eines Außenkreises bestimmt. Der Porendurchmesser entspricht dem Durchmesser des Außenkreises. Es wird über die Auswertung von mindestens 10 Poren gemittelt.

**Dicke der Schaumlage:** Die Dicke der Schaumlage wird an mindestens 5, gleichmäßig über die Probe verteilten Stellen mittels eines Dickenmessgeräts gemessen. Dabei ist zu gewährleisten, dass der Schaum durch das Messgerät nicht komprimiert wird.

**Dichte der Schaumlage:** Die Dichte wird ermittelt, indem eine Probe ausgeschnitten, gewogen und die Dicke ermittelt wird. Anschließend wird das Volumen durch Multiplikation von Dicke mit Fläche der Probe berechnet und schließlich das Gewicht durch das Volumen geteilt.

**Absorptionskapazität:** Für Absorptionsmessungen wird eine Testlösung, wie in BS EN 13726-1:2002 beschrieben, verwendet. Eine 25 cm² große Probe wird zuerst gewogen (W1), danach in die Testlösung getaucht und dort für mindestens eine Minute belassen. Anschließend wird die Probe vorsichtig an einer Ecke gefasst, ohne die Schaumlage zu quetschen, und für 10 Sekunden abtropfen gelassen. Danach wird erneut das Gewicht bestimmt (W2). Die Absorptionskapazität berechnet sich nun durch das Teilen der Betragsdifferenz von W2 und W1 durch das anfängliche Gewicht W1.

**Optische Differenzierung zwischen überspannten und nicht überspannten Makroporen:** Es wird die der adhäsiven Schicht zugewandte Oberfläche der Schaumlage mittels REM untersucht. Makroporen, welche eine Perforation (siehe Perforation der Sperrschicht) aufweisen, sind als nicht mit der Sperrschicht überspannt anzusehen.

**Perforation der Sperrschicht:** Eine Perforation wird nur als solche angesehen, falls ihr Durchmesser größer als 25 µm ist. Der Durchmesser wird durch Anlegen eines Außenkreises bestimmt. Der erhaltene Außenkreis entspricht dem Durchmesser der Perforation.

**Bestimmung des Anteils mit der Sperrschicht überspannter Makroporen:** Zur Bestimmung der Anzahl mit der Sperrschicht überspannter Makroporen wird die Oberfläche in Aufsicht mittels REM analysiert. Vorteilhafterweise wird eine Fläche von mindestens 25mm² untersucht. Nur teilweise sichtbare Poren, beispielsweise in den Randbereichen, werden nicht berücksichtigt. Sind in der Aufsicht mehrere Lagen an Poren erkennbar, so wird lediglich die oberste Lage berücksichtigt. Die Differenzierung zwischen überspannten und nicht überspannten Makroporen wird wie oben beschrieben durchgeführt. Die Anzahl überspannter Makroporen wird durch die Gesamtanzahl der berücksichtigten Makroporen dividiert.

**Bestimmung des Flächenanteils der Sperrschicht an der 2D-Oberfläche der Schaumlage:** Zur Bestimmung des Flächenanteils der Sperrschicht an der 2D-Oberfläche der Schaumlage wird die Oberfläche in Aufsicht rasterelektronenmikroskopisch analysiert. Vorteilhafterweise wird eine Fläche von mindestens 25 mm² (entspricht hier der 2D-Gesamtoberfläche) untersucht. Nur teilweise sichtbare Poren, beispielsweise in den Randbereichen, werden berücksichtigt. Die Fläche der Perforationen (≥ 25 µm) wird grafisch ermittelt und aufsummiert. Die Betragsdifferenz der Summe von der 2D-Gesamtoberfläche wird durch die 2D-Gesamtoberfläche geteilt.

**Bestimmung der Penetration des Silikongels:** Die Penetration des Silikongels wird in Anlehnung an DIN ISO 2137 durch die Tiefe des Einsinkens eines Konus (Gewicht 62,5 g) nach 60 Sekunden gemessen.

### Beispiele

Im Folgenden wird die Erfindung anhand mehrerer Beispiele näher erläutert.

### Beispiel 1: Herstellung einer Schaumlage mit Sperrschicht

Eine Wasserphase wird für die Schaumherstellung durch Lösen / Dispergieren des Tensids Pluronic F87 in einer Konzentration von 0,5 Gew% hergestellt. Gleichzeitig wird eine Teflonform mit einer ausreichenden Tiefe um einen Schaum von 7 mm Dicke mit Gießpapier ausgekleidet. Das Präpolymer Hypol 2001 wird in einer Konzentration von 40 Gew% zu der Wasserphase gegeben und bei Raumtemperatur mit einer Dispergierscheibe (1600 U/min) gemischt. Das erhaltene Gemisch wird sofort in die Gießform gegossen. Es wird gegen Luft geschäumt und für 10 Minuten ausgehärtet. Danach wird das Gießpapier entfernt und bei einer Temperatur von 150 °C für 3 Stunden getrocknet.

### Beispiel 2: Herstellen eines Silikonadhäsivs und Aufbringen des Adhäsivs auf Schaumlagen mit unterschiedlichem Anteil mit der Sperrschicht überspannter Makroporen

Es werden 3 verschiedene, 5 mm dicke Proben Schaumlagen mit unterschiedlichem Anteil mit der Sperrschicht überspannter Makroporen (Angabe in %) bereitgestellt.
- Probe 1: Freudenberg 3112 Oberseite (79%)
- Probe 2: Freudenberg 3112 Unterseite (98 %)
- Probe 3: Essentra Medisponge® SuperSoft™ 60P (3%)

Es wird vermutet, dass der unterschiedliche Anteil mit der Sperrschicht überspannter Makroporen beim Typ Freudenberg 3112 durch das Einwirken der Schwerkraft im Herstellungsprozess entsteht.

Eine Silikon-Adhäsiv-Vorläuferzusammensetzung wird durch Mischen der Komponenten Silpuran 2130 A und B im stöchiometrischen Verhältnis in einem Taumelmischer bei 2300 u/min für 1 min frisch hergestellt. Auf die vorgenannten Proben wird mit einer Handsprühdüse Perfekt 4 in Fließbecher-Ausführung der Firma Krautzberger mit einem 0,8 mm Sprühkopf bei 2,5 bar Luftdruck die flüssige Silikon-Adhäsiv-Vorläuferzusammensetzung aufgesprüht. Nach Aushärtung der Silikon-Adhäsiv-Vorläuferzusammensetzung für 5 min bei 100 °C wird die Adhäsion in Anlehnung an DIN EN 1939:2003 bestimmt.

Für Probe 1 mit 98 % Anteil mit der Sperrschicht überspannter Makroporen ergibt sich eine Adhäsion von *F̂*=0,13 N/2,5 cm bei einer vollflächigen Silikonadhäsivauflage von 30 g/m² und von *F̂*=0,08 N/2,5 cm bei einer Silikonadhäsivauflage von 10 g/m².

Für Probe 2 mit 79 % Anteil mit der Sperrschicht überspannter Makroporen ergibt sich eine Adhäsion von nur *F̂*=0,06 N/2,5 cm bei einer vollflächigen Silikonadhäsivauflage von 25 g/m².

Für Probe 3 mit 3 % Anteil mit der Sperrschicht überspannter Makroporen ist keine Adhäsion messbar bei einer vollflächigen Silikonadhäsivauflage von 30 g/m².

Es zeigt sich, dass mit einem höheren Anteil mit der Sperrschicht überspannter Makroporen auch bei geringerer Adhäsivauftragsmenge eine höhere Adhäsion erzielt werden kann.

### Beispiel 3: Nicht vollflächiges Aufbringen des Adhäsivs

Eine wie in Beispiel 2 beschrieben hergestellte Silikon-Adhäsiv-Vorläuferzusammensetzung wird mit einer Handsprühdüse Perfekt 4 in Fließbecher-Ausführung der Firma Krautzberger mit einem 0,8 mm Sprühkopf bei 2,5 bar Luftdruck auf Probe 1 aus Beispiel 2 aufgesprüht. Um ein regelmäßiges Muster aus Klebepunkten auf dem Schaum zu erzeugen, werden Schablonen mit normal-versetzter (hexagonaler) Rundlochung verwendet, die 1) 4,7 mm Lochdurchmesser und eine Teilung von 6,5 mm sowie 2) 2,6 mm Lochdurchmesser und eine Teilung von 3,8 mm aufweisen.
Mit beiden Schablonen wird ein mittlerer Schälwiderstand von *F̂*=0,08 N/2,5 cm bei einer Silikonadhäsivauflage von 10 g/m² auf dem Polyurethanschaum ermittelt.

### Kurzbeschreibung der Figuren

- Fig. 1: REM-Aufnahme einer Schaumlage in Aufsicht (erfindungsgemäß)
- Fig. 2: REM-Aufnahme der Sperrschicht in Aufsicht (erfindungsgemäß)
- Fig. 3: REM-Aufnahme der Schaumlage im Querschnitt (erfindungsgemäß)
- Fig. 4: REM-Aufnahme einer Schaumlage mit Folie als Barrierelage mit adhäsiver Schicht im Querschnitt (nicht erfindungsgemäß)
- Fig. 5: REM-Aufnahme einer Schaumlage ohne Sperrschicht mit adhäsiver Schicht im Querschnitt (nicht erfindungsgemäß)
- Fig. 6: Ineinandergreifendes Muster A
- Fig. 7: Ineinandergreifendes Muster B

### Figurenbeschreibung

Figur 1 zeigt eine REM-Aufnahme einer Schaumlage einer erfindungsgemäßen Hautauflage in Aufsicht. Es sind deutlich die mit der Sperrschicht überspannten Makroporen und eine Perforation der Sperrschicht zu erkennen.

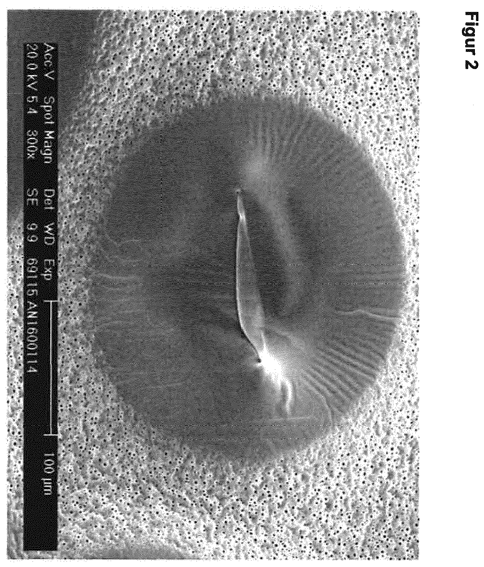

Figur 2 zeigt eine REM-Aufnahme der Sperrschicht einer erfindungsgemäßen Hautauflage in Aufsicht. Auf der Aufnahme sind eine mit der Sperrschicht überspannte Makropore und viele Mikroporen zu sehen.

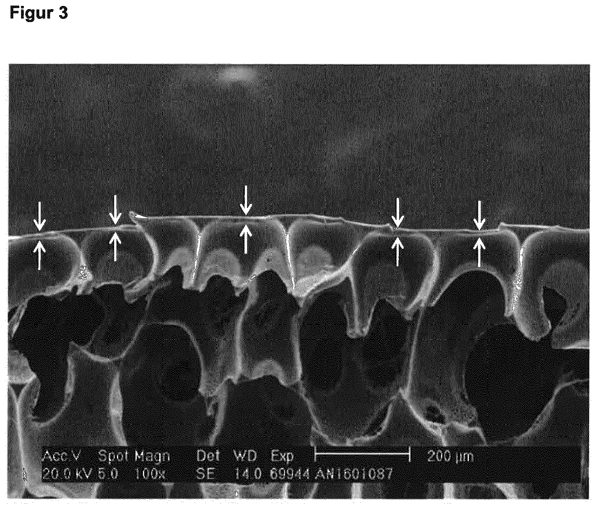

Figur 3 zeigt eine REM-Aufnahme der Schaumlage einer erfindungsgemäßen Hautauflage im Querschnitt. Mit weißen Pfeilen sind verschiedene Stellen zur Bestimmung der Dicke der Sperrschicht gekennzeichnet.

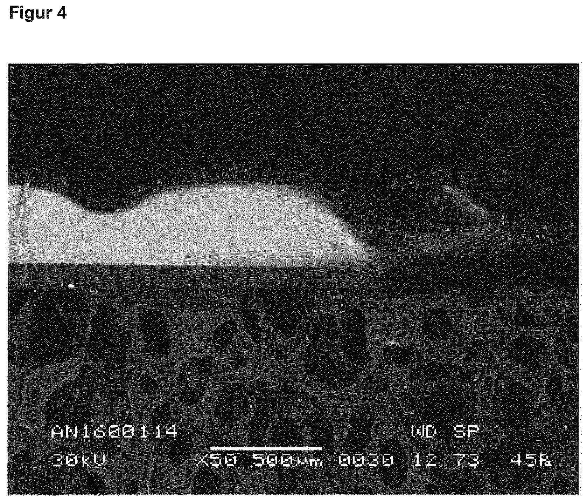

Figur 4 zeigt eine REM-Aufnahme einer Schaumlage mit Folie als Barrierelage mit adhäsiver Schicht im Querschnitt (nicht erfindungsgemäß). Die adhäsive Schicht wurde hier mittels eines Transferbeschichtungsverfahrens aufgebracht.

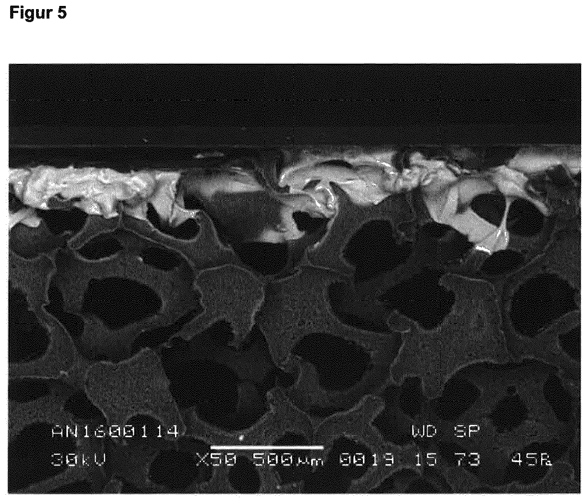

Figur 5 zeigt eine REM-Aufnahme einer Schaumlage ohne Sperrschicht mit adhäsiver Schicht im Querschnitt (nicht erfindungsgemäß). Man erkennt, dass die adhäsive Schicht in erheblichem Maße in die Schaumlage eingedrungen ist.

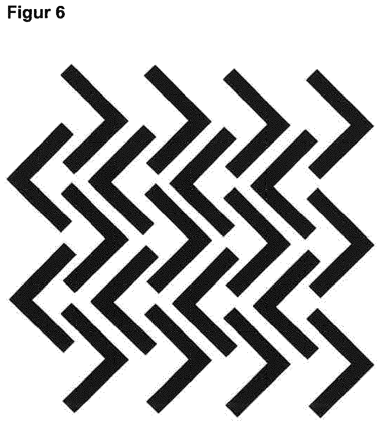

Figur 6 zeigt ein ineinandergreifendes Muster, welches eine laterale Ausbreitung von Wundexsudat verhindern kann.

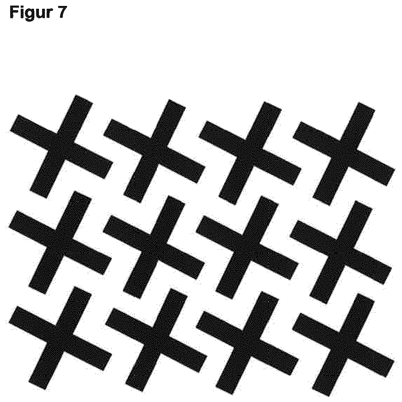

Figur 7 zeigt ein weiteres, ineinandergreifendes Muster, welches eine laterale Ausbreitung von Wundexsudat verhindern kann.

## Patentansprüche

1. Hautauflage umfassend eine offenzellige Schaumlage und eine darauf angeordnete, für den Hautkontakt vorgesehene, adhäsive Schicht, **dadurch gekennzeichnet, dass** zumindest die der adhäsiven Schicht zugewandte Seite der Schaumlage Makroporen aufweist, deren Kavitäten zumindest anteilig durch eine aus der Schaumlage gebildete Sperrschicht überspannt sind **dadurch gekennzeichnet, dass** der Flächenanteil der Sperrschicht an der Oberfläche der Schaumlage mindestens 20 % beträgt und/oder dass mindestens 20 % der an die Oberfläche der Schaumlage angrenzenden Makroporen durch die Sperrschicht überspannt sind.

2. Hautauflage nach Anspruch 1 **gekennzeichnet durch** eine Absorptionskapazität von mindestens 5 g/g.

3. Hautauflage nach einem oder mehreren der vorgenannten Ansprüche, **dadurch gekennzeichnet, dass** die Sperrschicht Mikroporen aufweist.

4. Hautauflage nach einem oder mehreren der vorgenannten Ansprüche, **dadurch gekennzeichnet, dass** die Sperrschicht planar ist.

5. Hautauflage nach einem oder mehreren der vorgenannten Ansprüche, **dadurch gekennzeichnet, dass** die Dicke der Sperrschicht weniger als 100 µm beträgt.

6. Hautauflage nach einem oder mehreren der vorgenannten Ansprüche, **dadurch gekennzeichnet, dass** die adhäsive Schicht eine Dicke von weniger als 200 µm aufweist und/oder in einer Auftragsmenge von weniger als 200 g/m² vorliegt.

7. Hautauflage nach einem oder mehreren der vorgenannten Ansprüche, **dadurch gekennzeichnet, dass** die adhäsive Schicht unmittelbar auf der Oberfläche der Schaumlage angeordnet ist.

8. Hautauflage nach einem oder mehreren der vorgenannten Ansprüche, **dadurch gekennzeichnet, dass** die Hautauflage auch bei Auftragsmengen von weniger als 200 g/m² Spitzenschälwiderstände gegen ein Stahlsubstrat von mehr als 0,05 N/2,5 cm aufweist.

9. Hautauflage nach einem oder mehreren der vorgenannten Ansprüche, **dadurch gekennzeichnet, dass** die adhäsive Schicht die Schaumlage nur teilweise bedeckt und/oder die Oberfläche der Schaumlage nur teilweise von der Sperrschicht gebildet ist.

10. Hautauflage nach einem oder mehreren der vorgenannten Ansprüche, **dadurch gekennzeichnet, dass** die adhäsive Schicht und/oder Sperrschicht als unzusammenhängendes und/oder ineinandergreifendes Muster vorliegt.

11. Hautauflage nach einem oder mehreren der vorgenannten Ansprüche, **dadurch gekennzeichnet, dass** die adhäsive Schicht Silikone, insbesondere Silikongele, Silikonelastomere und/oder mit Substituenten wie Polyethylenglykol und/oder Polyurethan modifizierte quervernetzte Polyorganosiloxane enthält.

12. Hautauflage nach einem oder mehreren der vorgenannten Ansprüche, **dadurch gekennzeichnet, dass** die Schaumlage einen hydrophilen Polymerschaum, insbesondere einen Polyurethanschaum, umfasst.

13. Hautauflage nach einem oder mehreren der vorgenannten Ansprüche zur nicht-medizinischen Regulierung der Feuchtigkeit in Hautnähe, zur Erzeugung eines feuchten Wundklimas und mithin zur feuchten Wundbehandlung, insbesondere von chronischen Wunden, und/oder zur Verhinderung von Druckgeschwüren.

14. Verfahren zur Herstellung der Hautauflage nach einem oder mehreren der vorgenannten Ansprüche, umfassend die folgenden Schritte:
(A) Herstellen und/oder Bereitstellen einer Schaumlage, die zumindest auf einer Seite Makroporen aufweist, deren Kavitäten zumindest anteilig durch eine aus der Schaumlage gebildete Sperrschicht überspannt sind;
(B) auftragen eines adhäsiven Materials und/oder Vorläuferverbindungen hiervon auf die zumindest eine Seite der Schaumlage, die die Sperrschicht aufweist;
(C)gegebenenfalls Aushärten des adhäsiven Materials und/oder Vorläuferverbindungen hiervon unter Ausbildung einer adhäsiven Schicht.

15. Verfahren nach Anspruch 14, **dadurch gekennzeichnet, dass** das adhäsive Material und/oder Vorläuferverbindungen hiervon durch Sprühen, Walzenauftrag, insbesondere Roll Kiss Coating, und/oder Schlitzdüsenauftrag aufgebracht wird.

## Claims

1. Skin contact material comprising an open-celled foam ply and an adhesive layer disposed thereon and intended for skin contact, **characterized in that** at least that side of the foam ply that faces the adhesive layer has macropores, the cavities of which are spanned at least proportionally by a barrier layer formed from the foam ply, **characterized in that** the area of the barrier layer as a proportion of the surface of the foam ply is at least 20% and/or in that at least 20% of the macropores bordering the surface of the foam ply are spanned by the barrier layer.

2. Skin contact material according to Claim 1, **characterized by** an absorption capacity of at least 5 g/g.

3. Skin contact material according to one or more of the preceding claims, **characterized in that** the barrier layer has micropores.

4. Skin contact material according to one or more of the preceding claims, **characterized in that** the barrier layer is planar.

5. Skin contact material according to one or more of the preceding claims, **characterized in that** the thickness of the barrier layer is less than 100 µm.

6. Skin contact material according to one or more of the preceding claims, **characterized in that** the adhesive layer has a thickness of less than 200 µm and/or is present at an application rate of less than 200 g/m².

7. Skin contact material according to one or more of the preceding claims, **characterized in that** the adhesive layer is disposed directly on the surface of the foam ply.

8. Skin contact material according to one or more of the preceding claims, **characterized in that** the skin contact material, even at application rates of less than 200 g/m², has peak peel resistances against a steel substrate of more than 0.05 N/2.5 cm.

9. Skin contact material according to one or more of the preceding claims, **characterized in that** the adhesive layer covers the foam ply only partly and/or the surface of the foam ply is only partly formed by the barrier layer.

10. Skin contact material according to one or more of the preceding claims, **characterized in that** the adhesive layer and/or barrier layer takes the form of an incoherent and/or interengaging pattern.

11. Skin contact material according to one or more of the preceding claims, **characterized in that** the adhesive layer comprises silicones, more particularly silicone gels, silicone elastomers and/or crosslinked polyorganosiloxanes modified with substituents such as polyethylene glycol and/or polyurethane.

12. Skin contact material according to one or more of the preceding claims, **characterized in that** the foam ply comprises a hydrophilic polymer foam, more particularly a polyurethane foam.

13. Skin contact material according to one or more of the preceding claims for non-medical regulation of the moisture near to the skin, for generating a moist wound climate and therefore for moist wound treatment, more particularly of chronic wounds, and/or for prevention of pressure sores.

14. Method for producing the skin contact material according to one or more of the preceding claims, comprising the following steps:
(A) producing and/or providing a foam ply which at least on one side has macropores whose cavities are spanned at least proportionally by a barrier layer formed from the foam ply;
(B) applying an adhesive material and/or precursor compounds thereof to the at least one side of the foam ply that has the barrier layer;
(C) optionally curing the adhesive material and/or precursor compounds thereof to form an adhesive layer.

15. Method according to Claim 14, **characterized in that** the adhesive material and/or precursor compounds thereof are applied by spraying, roll application, more particularly roll kiss coating, and/or slot die application.

## Revendications

1. Coussinet cutané comprenant une nappe de mousse à cellules ouvertes et une couche adhésive disposée pardessus et destinée au contact avec la peau, **caractérisé en ce qu'**au moins le côté de la nappe de mousse qui est dirigé vers la couche adhésive comporte des macropores dont les cavités sont au moins partiellement recouvertes par une couche barrière formée à partir de la nappe de mousse, **caractérisé en ce que** la proportion de surface de la couche barrière sur la surface de la nappe de mousse est d'au moins 20 % et/ou **en ce qu'**au moins 20 % des macropores adjacents à la surface de la nappe de mousse sont recouverts par la couche barrière.

2. Coussinet cutané selon la revendication 1, **caractérisé par** une capacité d'absorption d'au moins 5 g/g.

3. Coussinet cutané selon une ou plusieurs des revendications précédentes, **caractérisé en ce que** la couche barrière comporte des micropores.

4. Coussinet cutané selon une ou plusieurs des revendications précédentes, **caractérisé en ce que** la couche barrière est plane.

5. Coussinet cutané selon une ou plusieurs des revendications précédentes, **caractérisé en ce que** l'épaisseur de la couche barrière est inférieure à 100 µm.

6. Coussinet cutané selon une ou plusieurs des revendications précédentes, **caractérisé en ce que** la couche adhésive a une épaisseur inférieure à 200 µm et/ou est présente dans une quantité d'application inférieure à 200 g/m².

7. Coussinet cutané selon une ou plusieurs des revendications précédentes, **caractérisé en ce que** la couche adhésive est disposée directement sur la surface de la nappe de mousse.

8. Coussinet cutané selon une ou plusieurs des revendications précédentes, **caractérisé en ce que** le coussinet cutané présente une résistance au pelage de pointes contre un substrat en acier de plus de 0,05 N/2,5 cm même avec des quantités d'application inférieures à 200 g/m².

9. Coussinet cutané selon une ou plusieurs des revendications précédentes, **caractérisé en ce que** la couche adhésive ne recouvre que partiellement la nappe de mousse et/ou la surface de la nappe de mousse n'est que partiellement formée par la couche barrière.

10. Coussinet cutané selon une ou plusieurs des revendications précédentes, **caractérisé en ce que** la couche adhésive et/ou la couche barrière se présente sous la forme d'un motif incohérent et/ou emboîtable.

11. Coussinet cutané selon une ou plusieurs des revendications précédentes, **caractérisé en ce que** la couche adhésive contient des silicones, notamment des gels de silicone, des élastomères de silicone et/ou des polyorganosiloxanes à réticulation transversale modifiés par des substituants tels que le polyéthylène glycol et/ou le polyuréthane.

12. Coussinet cutané selon une ou plusieurs des revendications précédentes, **caractérisé en ce que** la nappe de mousse comprend une mousse de polymère hydrophile, en particulier une mousse de polyuréthane.

13. Coussinet cutané selon une ou plusieurs des revendications précédentes destiné à la régulation non médicale de l'humidité au voisinage de la peau, à la génération d'un climat de plaies humide et donc au traitement de plaies humide, en particulier de plaies chroniques, et/ou à la prévention d'escarres.

14. Procédé de fabrication du coussinet cutané selon une ou plusieurs des revendications précédentes, le procédé comprenant les étapes suivantes :
(A) fabriquer et/ou fournir une nappe de mousse qui comporte sur au moins un côté des macropores dont les cavités sont au moins partiellement couvertes par une couche barrière formée à partir de la nappe de mousse ;
(B) appliquer une matière adhésive et/ou des composés précurseurs de celle-ci sur au moins un côté de la nappe de mousse qui comporte la couche barrière ;
(C) si nécessaire, faire durcir la matière adhésive et/ou ses composés précurseurs avec formation d'une couche adhésive.

15. Procédé selon la revendication 14, **caractérisé en ce que** la matière adhésive et/ou ses composés précurseurs sont appliqués par pulvérisation, application au rouleau, en particulier revêtement Kiss Roll, et/ou application par buse à fente.
